# EUROPEAN PATENT APPLICATION

(11) **EP 4 282 445 A1**
(43) Date of publication of application: **29.11.2023**
(21) Application number: 22175821.2
(22) Date of filing: 27.05.2022
(51) Int. Cl.: A61L 27/26, A61L 27/38, A61L 27/44, A61L 27/54, A61L 27/56

(54) **AN ANTI-INFLAMMATORY THREE-DIMENSIONAL CONSTRUCT AND METHOD OF MANUFACTURING**

(71) Applicant: Lietuvos sveikatos mokslu universitetas, 44307 Kaunas (LT)
(72) Inventor: Maciulaitis, Justinas, LT-44307 Kaunas (LT); Maciulaitis, Romaldas, LT-44307 Kaunas (LT); Gudas, Rimtautas, Kaunas (LT)
(74) Representative: Pakeniene, Ausra

(57) **Abstract**

The invention is immunomodulatory cell seeded three-dimensional construct containing anti-inflammatory properties, comprising electrospinned polycaprolactone (PCL) material supplemented with polylactic-glicolic acid (PLGA). The construct is seeded with mesenchymal stem cells. The construct is augmented with nicotinamide particles, for anti-inflammatory properties, which complement immunomodulatory properties exhibited by the mesenchymal stem cells.

The augmented construct is characterized by anti-inflammatory and biocompatibility properties for cartilage tissue engineering during the construct degradation after implantation within inflammatory joint.

## Description

### TECHNICAL FIELD

The invention relates to immunomodulatory cell seeded three-dimensional constructs having anti-inflammatory properties for degenerative cartilage regeneration.

### BACKGROUND ART

Osteoarthritis (OA) affects 20% of the population and is the most common orthopedic disorder. 40% of people over 60 have OA, usually the knee joint [1]. In 95% of cases, the affected cartilage has a very limited or no possibility of recovery, which causes varying degrees of disability for the elderly. This poses significant health, social and economic challenges.

OA can damage all human joints, cartilage deformities, fibrillation, and cracks. Rehabilitation, physiotherapy procedures, anti-inflammatory drugs, platelet-rich plasma and endoprostheses are used to treat degenerative cartilage disease. The methodologies and technologies used to treat this disease often differ between different scientific and clinical groups. Biological therapy is becoming increasingly important in the treatment of cartilage damage caused by osteoarthritis. However, different cells, media, sera, growth factors are used, which differ between manufacturers, and the raw material used by different manufacturers affects the variability of the final product.

A local osteoarthritic environment in the joint is typically described as inflammatory. Therefore, injected/ implanted therapeutic agent is immediately under inflammatory stress, thus impairing the possible regenerative potential. Sustained inflammation negatively affects cell growth, proliferation and tissue regeneration.

Tissue engineered construct (TEC) biocompatibility is highly dependent on scaffold biomechanical and physical features in vitro [1]. Customization of scaffolds with highly predictable physical characteristics, such as mechanical rigidity, pore size, porosity and pore interconnectivity are enabled by the ability to adjust parameters of synthetic polymeric feed material [6].

These critical structural parameters provide a homogenous distribution of cells and nutrient transfer within the scaffold [2]. The ability of chondrocytes to effectively adhere to the scaffold is highly dependent on cell viability. In addition, viable cell proliferation is indicative of the ability to deposit ECM on the scaffold, thus identification of optimal biomaterial morphological parameters is essential for specific cellular processes [3].

Foreign biomaterials can cause significant inflammation for patients when implanted in vivo. This in turn, affects tissue repair processes and functional integration of the biomaterial. Thus, in addition to structural repair of the tissue, immunomodulation of inflammatory response is necessary to enable a favorable regenerative effect [4].

Studies have been employed previously to improve immunomodulatory properties of the scaffold. This has been achieved by incorporating immunomodulatory and anti-inflammatory organic and inorganic molecules. (Lewis et al).

Polycaprolactone - IUPAC name Poly (hexane-6-lactone) is a biodegradable polyester with a low melting point (about 60 ° C). This polymer is resistant to mechanical impact. Polycaprolactone is biodegradable and biocompatible. In humans, caprolactone is biodegraded by hydrolysis of ester linkages [5].

However, the synthetic biodegradable polymers have been reported to cause the acidification of the cartilage tissue. They also induced loss of chondrogenic phenotype and the inflammatory responses in vitro [6].

PLGA degradation/ hydrolysis in vivo, causes acidification in the vicinity of the scaffold, thus resulting in the cell toxicity and local inflammation [10]. After the degradation, lactic and glycolic acid are formed as by-products, which in turn negatively affect cell growth, proliferation and tissue regeneration.

Nicotinic acid is a stable and water-soluble vitamin. It is a known and inexpensive compound that is known to modulate the activity of various types of immune cells, such as macrophages, dendritic cells, neutrophils, and lymphocytes.

Cells are the active substance of the tissue engineered product that when combined with cells, forms a construct. A subset of mesenchymal stem cells (MSC) has been frequently used as an active substance for tissue engineering, including cartilage tissue [7]. MSCs hold the potential for self-renewal differentiation into multiple lineages, and immunomodulatory properties, thus making them attractive candidates for cartilage tissue engineering. Paracrine activity of the cells could have augmented effect on superior immunomodulation [8].

European patent application No. EP 18215447.6 (publication No. EP3533476A1) describes a method for making a porous devitalized scaffold and a scaffold suitable for use in repair of osseous, chondral, or osteochondral defects in a mammal comprises the steps of providing micronized extracellular matrix (ECM) tissue, mixing the micronized extracellular matrix with a liquid to provide a slurry, and freeze-drying the slurry to provide the porous scaffold. A porous scaffold suitable for use in repair of osseous, chondral, or osteochondral defects in a mammal and comprising a porous freeze-dried matrix formed from micronized decellularized extracellular matrix tissue is also described. However, the scaffold does not address the inflammatory in vivo condition and prevents for quality osteoarthritic cartilage regeneration. Moreover, the scaffold does not address durability and biocompatibility issues of such scaffolds. Also, the continuous inflammation in the joint during osteoarthritic cartilage degeneration is not addressed.

Zamanlui et al [9] tested PLGA/PCL blend for bone marrow stem cells chondrogenic differentiation. It showed the positive chondrogenic-determined differentiation, however the construct does not address the inflammatory in vivo condition and subsequent scaffold degradation which causes detrimental acidification in the joint.

The present invention is dedicated to overcoming lack of anti-inflammatory and biocompatibility properties of a construct used for cartilage tissue engineering during the immunomodulatory cell seeded three-dimensional construct containing anti-inflammatory properties degradation after implantation in inflammatory joint and for producing further advantages.

### SUMMARY OF INVENTION

The invention is immunomodulatory cell seeded three-dimensional construct containing anti-inflammatory properties, comprising electrospinned polycaprolactone (PCL) material supplemented with polylactic-glicolic acid (PLGA). The construct is seeded with mesenchymal stem cells. The construct is augmented with nicotinamide particles, for anti-inflammatory properties, which complement immunomodulatory properties exhibited by the mesenchymal stem cells.

The augmented construct is characterized by anti-inflammatory and biocompatibility properties for cartilage tissue engineering during the construct degradation after implantation within inflammatory joint.

### BRIEF DESCRIPTION OF THE DRAWINGS

Features of the invention believed to be novel and inventive are set forth with particularity in the appended claims. The invention itself, however, may be best understood by reference to the following detailed description of the invention, which describes exemplary embodiments, given in non-restrictive examples, of the invention, taken in conjunction with the accompanying drawings, in which:
Preferred embodiments of the invention will be described herein below with reference to the drawings. Each figure contains the same numbering for the same or equivalent element.

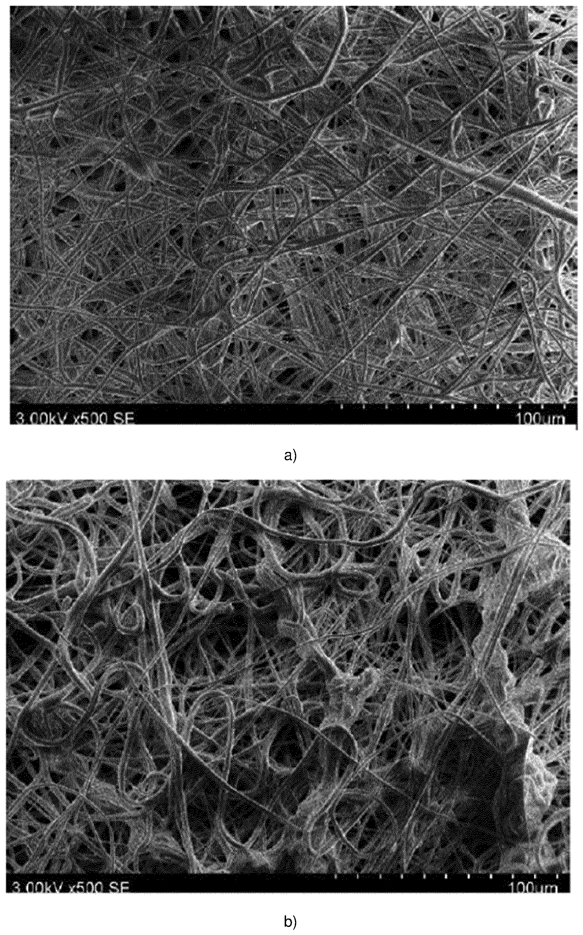

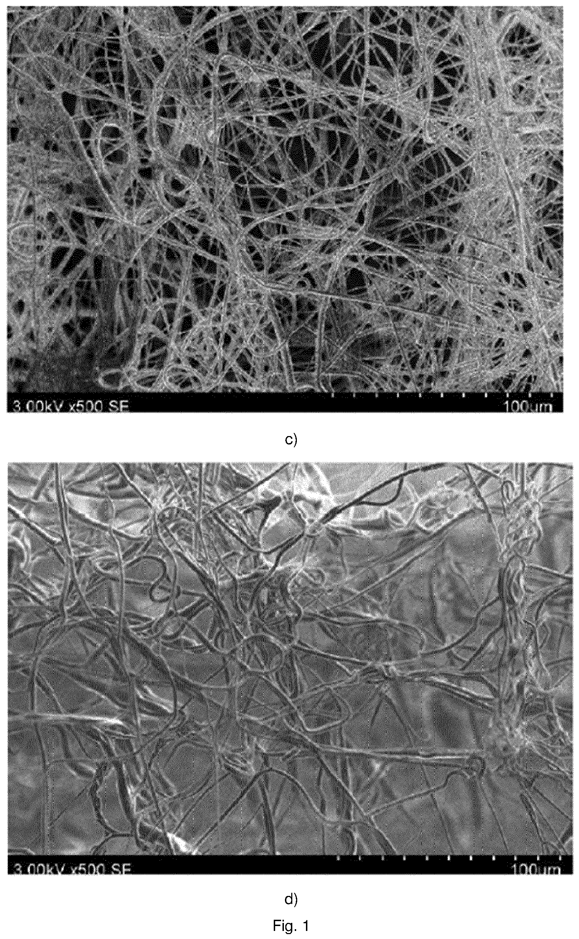

Fig. 1 shows scanned electron microscopy images of examples of constructs according to the invention:
   a) PCL:PLGA - 3:1 (wt/wt) - construct with added PCL:PLGA, but without added Nicotinamide, a control scaffold;
   b) PCL:PLGA - 3:1 (wt/wt) + Nicotinamide 1% - construct with added PCL:PLGA and Nicotinamide at 1%;
   c) PCL:PLGA - 3:1 (wt/wt) + Nicotinamide 3% - construct with added PCL:PLGA and Nicotinamide at 3%;
   d) PCL:PLGA - 3:1 (wt/wt) + Nicotinamide 6% - construct with added PCL:PLGA and Nicotinamide at 6%.
Fig. 2 shows contact angle between the construct-water interface: Fig. 2a construct a), Fig. 2b, construct b), in Fig. 2c construct c), in Fig. 2d construct d) of Fig.1 according to the invention.
Fig. 3 shows FTIR spectra in: a) construct PCL:PLGA - 3:1 (wt/wt), b) PCL:PLGA - 3:1 (wt/wt) + Nicotinamide 1%, c) PCL:PLGA - 3:1 (wt/wt) + Nicotinamide 3%, d) PCL:PLGA - 3:1 (wt/wt) + Nicotinamide 6%
Fig. 4. shows Nicotinamide release kinetics. The increasing amount of nicotinamide is released with increasing concentrations of the molecule per construct according to the invention. Values of cumulative nicotinamide release are presented in Table 1.

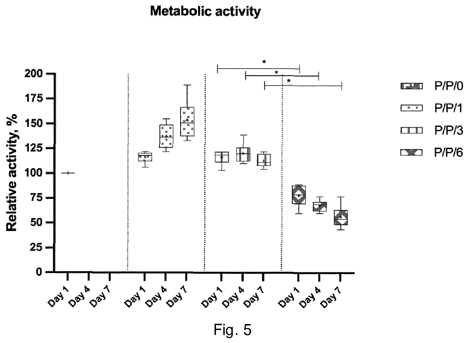

Fig. 5. Shows the metabolic activity of the seeded mesenchymal stem cells on the construct according to the invention. The higher initial metabolic activity in the 1% construct, is better retained at later days of culture, at least up to day 7. Values of metabolic activity are presented in Table 2.

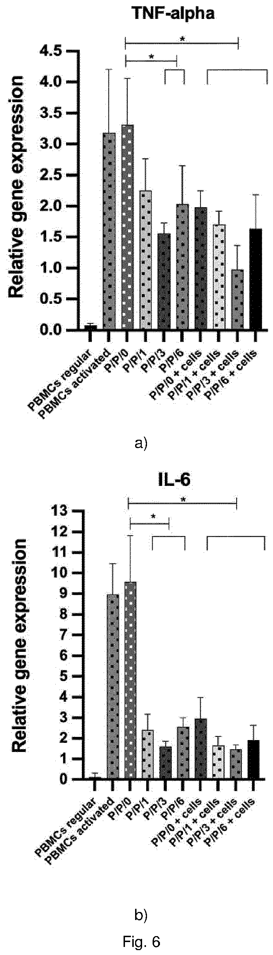

Fig. 6. Shows the addition of cells significantly improved TNF expression suppressive capabilities in all groups tested. The cell treated groups were significantly superior compared to unloaded P/P/0, example shown in Fig.1a), construct. A clear tendency for the superior 3% loaded construct amongst the groups was evident, as revealed by the gene expression and protein values. All nicotinamide treated construct groups diminished IL-6 expression, and addition of cells only improved the untreated P/P/0 group. Values of gene expression profiles are presented in Table 3.

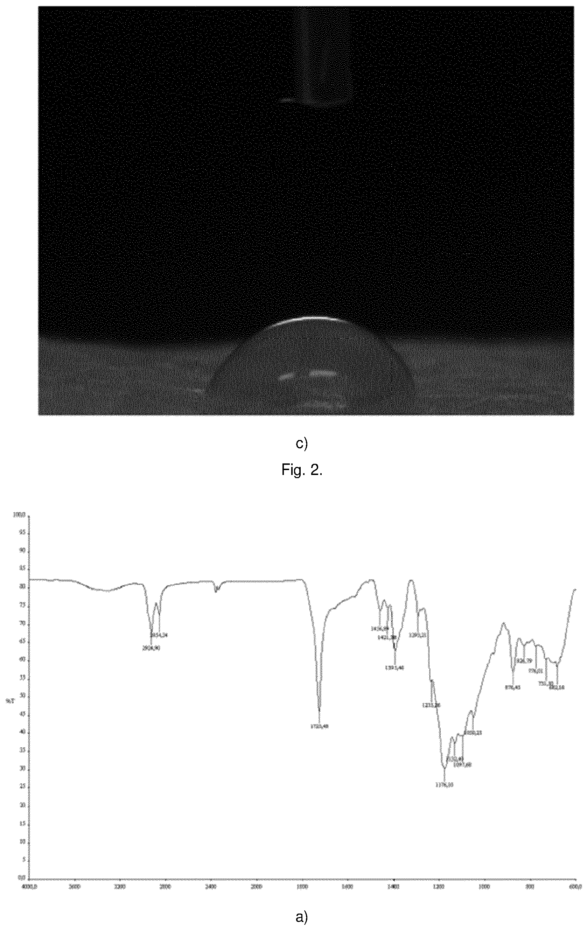

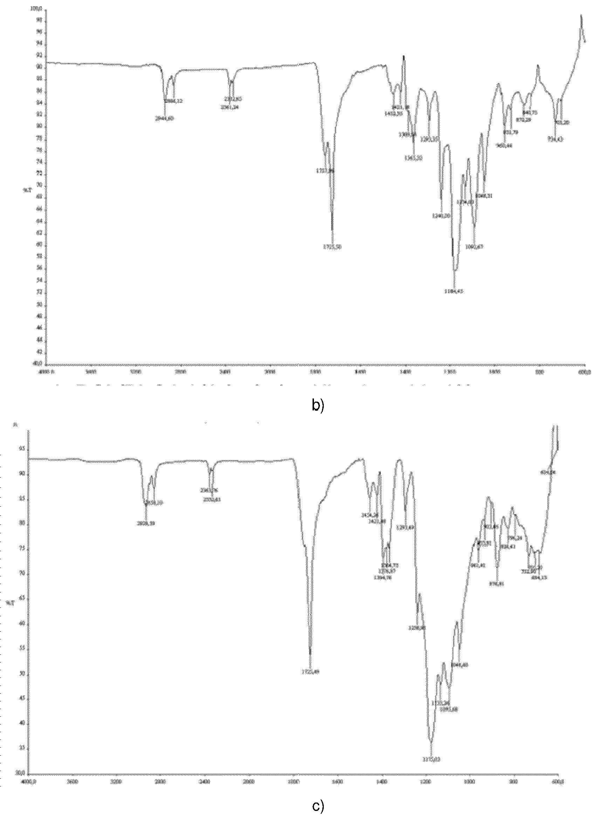

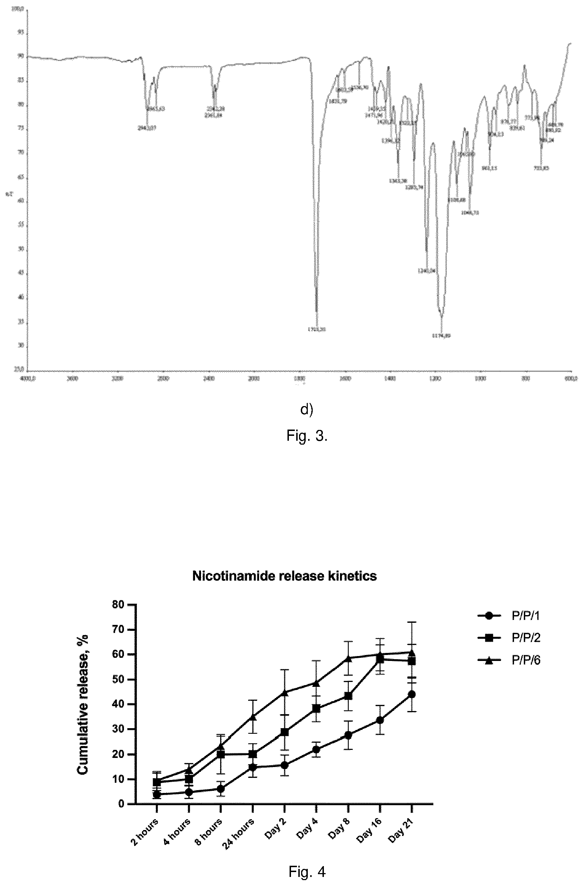

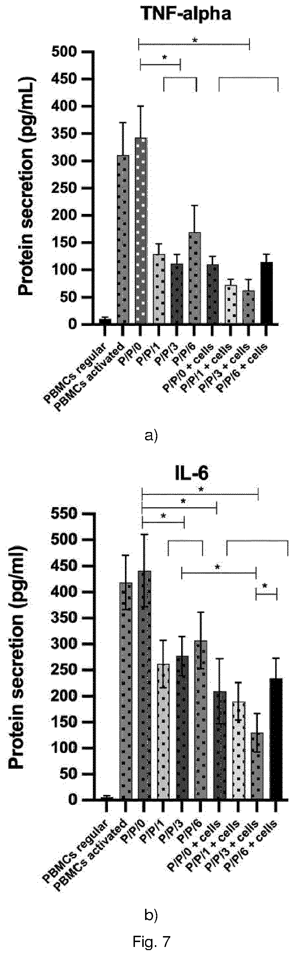

Fig. 7. Nicotinamide treated construct had superior TNF-alpha protein secretion suppressing capabilities. Untreated P/P/0, example shown in Fig.1a), construct did not have the same positive properties. However, addition of cells highly improved potency to diminish inflammation in all groups tested. Nicotinamide treated construct had superior TNF-alpha protein secretion suppressing capabilities untreated P/P/0 construct did not have the same positive properties. Cells addition to the construct significantly improved anti-inflammatory properties in P/P/0, example shown in Fig.1a), and P/P/3, example shown in Fig.3a), groups. P/P/6, example shown in Fig.1d), had an inferior anti-inflammatory property and was not able to reduce protein secretion, compared to P/P/3 group. Values of protein release are presented in Table 4.

### DETAILED DESCRIPTION OF THE INVENTION

It should be understood that numerous specific details are presented to provide a complete and comprehensible description of the invention embodiment. However, the person skilled in art will understand that the embodiment examples do not limit the application of the invention which can be implemented without these specific instructions. Well-known methods, procedures and components have not been described in detail for the embodiment to avoid misleading. Furthermore, this description should not be considered to be constraining the invention to given embodiment examples but only as one of possible implementations of the invention.

Method of producing an anti-inflammatory three-dimensional cellular construct for reconstruction of symptomatic, local cartilage defects, according to the invention, comprises use of polycaprolactone (PCL) material for making scaffold by electrospinning method.

Method of producing a bioactive three-dimensional scaffold according to the invention, further comprises selecting and using poly-D-L-lactide-co-glycolide (PLGA) with a lactic acid monomer content of 75% in the polymer and a glycolic acid monomer content of 25%, maintaining a monomer ratio of 75:25. This imparts superior biocompatibility properties to the scaffold because of PCL and the retained mechanical and tensile strength because of the PLGA content.

PCL/PLGDA blend improves biocompatibility of the construct.

Incorporation of nicotinamide particles allow to overcome in vivo joint inflammatory status.

The added mesenchymal stem cells greatly improve cartilage regeneration capabilities of the bioactive three-dimensional cellular construct for reconstruction of symptomatic, osteoarthritic cartilage defects, by suppressing local inflammation in the joint. Perinatal mesenchymal stem cells we used to assess the biocompatibility of the prepared constructs. To evaluate the cytocompatibility of the prepared constructs and the viability of the seeded cells, a Presto Blue viability test was carried out. The Presto blue assay was performed at a predetermined time (1, 2, 3, and 4 days).

Method of producing a three-dimensional cellular construct for reconstruction of symptomatic, local cartilage defects preferably comprise combining Polycaprolactone, PLGA, Nicotinamide at 1% concentration and mesenchymal stem cells.

The method of producing immunomodulatory cell seeded three-dimensional cellular construct containing anti-inflammatory properties for degenerative cartilage regeneration comprises:
- scaffold preparation step,
- construct preparation steps.

Examples of constructs according to the invention prepared by the method according to the invention:
- PCL / PLGA 3/1 (wt / wt);
- PCL / PLGA 3/1 (wt / wt) + Nicotinic acid 1% (By weight of PLGA);
- PCL / PLGA 3/1 (wt / wt) + Nicotinic acid 3% (By weight of PLGA);
- PCL / PLGA 3/1 (wt / wt) + Nicotinic acid 6% (By weight of PLGA).

Preferably Polycaprolactone (PCL) of mol wt 80,000 and PLGA- Poly(D,L-lactide-co-glycolide) lactide:glycolide (ratio 75:25), mol wt 66,000-107,000 are used.

Preferably Nicotinamide particles were used as functional agents for the formation of tissue. Nicotinamide concentration of 0-6%, preferably at a concentration of 1%.

Preferably acetone and dimetilformamide solvents are used in preparation of polymer solution. Preferably the polymer solution concentration is at 20% wt/v. Preferably solvent ratio of acetone:dimetilformamide is 2:3 v/v. For example, to create a solution of 10 ml, 4ml of acetone and 6 ml of dimetilformamide 6 ml (ACE/DMF, 2/3, v/v) are used. Polymer is mixed by combining 1,5 g PCL with 0,5 g PLGA, total at 2 g (PCL/PLGA, 3/1, wt/wt) of polymer.

Electrospinning parameters for electrospinning a scaffold according to the invention are preferably as follows: polymer solution supply flow 2.3 ml/h; needle no. 21; distance between needle and collector 15 cm; voltage between needle and collector 22 kV; ambient temperature 30°C; relative humidity 40%. Aluminum foil substrate. The needle moves in the x-axis at 5 cm intervals. The polymer solution is supplied with a Teflon hose. As an example, one sample is formed from 10 ml solution. Dry ice is added to the collector every 25 minutes.

Constructs after electrospinning are lyophilized by freezing the samples in a freezer at -20°C for 24 h. Frozen constructs were lyophilized under a vacuum of 2 × 10⁻⁶ mPa at -40°C for 72 h.

Preferably, constructs after lyophilization are stored in glass airtight vials in the dark at 20-25°C and 40-60% relative humidity.

The morphology of a sample of constructs was analyzed using scanning electron microscopy (SEM S-3400N, Hitachi Ltd, Japan). Analysis of magnesium and oxygen atoms was performed by electron dispersive X-ray spectroscopy (EDS) using the same equipment as SEM at a voltage of 15 kV and a working distance of 10 mm. SEM photographs of the developed and manufactured examples of constructs according to the invention are presented in Table 1. Constructs comprises a microfiber structure. The strands are arranged in a random order, the strands are not oriented in one direction.

Chemical changes in construct samples were analyzed using the Fourier Transform Infrared (FTIR) spectra (Perkin-Elmer Frontier, USA) in the range of 4000 to 650 cm⁻¹.

SEM photographs of the developed and manufactured construct samples are shown in Figures 1a) - Fig. 1-d). From the SEM photographs it is evident that the layout of the constructs consists of a dispersed microfiber structure. In all layout samples, the strands are arranged in a random order, the strands are not oriented in one direction.

Mechanical properties of the examples of the constructs according to the invention:
Mechanical properties of construct samples were determined using a universal material testing machine (BDO-FB 0.5 TH, Zwick GmbH & Co, Germany) in a controlled environment according to ASTM D 882 standard. Rectangular samples with a length of 60 mm, a width of 14 mm and a thickness of 0.15 mm were used for the measurements. Tensile force measurements of the developed examples of constructs according to the invention are shown in Table 2. Sample No. 1, which consisted only of a mixture of PCL and PLGA 3/1, had a tensile force of 77 ± 6 Pa. The tensile force of the samples with nicotinic acid additive ranged from 65 ± 7 Pa to 140 ± 28 Pa.

Hydrophilicity of the examples of the constructs according to the invention:
The hydrophilicity of the construct samples was measured using a Theta Lite TL 101 optical tensiometer (Biolin Scientific, Finland).

The wetting angle results of the scaffold no1. made of a mixture of PCL and PLGA was 64.7°. The addition of nicotinic acid had virtually no effect on the water wetting angle of the samples, which was 63.8° (1% nicotinic acid fraction), 62.2° (3%) and 62.7° (6%). All designed and manufactured models have hydrophilic properties. The hydrophilicity is depicted in Fig. 3a-3d1.

### Functional groups of the surface of layout threads:

The FTIR spectra of the developed and manufactured models are presented in Figures 4a-4d.

This analysis allows to determine the presence of functional groups on the surface of the layout threads.

FTIR spectra of all constructs have peaks in the range 3000-3700 cm-1, which are characteristic of -nicotinamide functional group fluctuations. This peak is broad in nature with low intensity, it is characteristic of the nicotinamide peak in the FTIR spectrum.

### Construct preparation:

All experimental procedures were approved and conducted according to the standard guidelines and protocols set by the Kaunas Regional Biomedical Research Ethics Committee. An informed signed content was obtained from the patient. Placental tissue was collected during routine cesarean section surgery procedure as a waste material. Briefly, a tissue was washed extensively, and digested with collagenase. Isolated cells were plated and cultured in culture medium supplemented with fetal bovine serum, gentamicin at 37 °C in a humid atmosphere with 5% CO₂. Cells were harvested when reached 80-90% confluence.

Constructs were soaked in proliferation medium one day before seeding.

Characteristics of the construct according to the invention are as follows:
comprises a cylindrical shape, preferably having diameter of 6 mm and a height of 1 mm;
morphological composition of the scaffolds comprises a microfiber structure in which filaments are arranged in a random direction, i.e. non-oriented;
average filament diameter in the scaffolds is preferably in the range from 1×10⁻⁶ to 1×10⁻⁵ m;
statistical dispersion of scaffolds' filament thickness is preferably 90% variance;
scaffolds' filament thread length is preferably 90th percentile value 6×10⁻⁵; m;
scaffolds' porosity is preferably 85%;
mechanical tensile properties of the scaffolds are preferably 0.6 Pa;
hydrophilicity of the scaffolds is preferably wetting angle 70°;
Surface of the scaffolds comprises hydroxyl groups attached thereto.

In the final step the examples of constructs according to the invention were seeded with cells. Cell content on one scaffold is restricted to 1×10⁶ cells to 1 cm² area of the scaffold. After trypsinization with Tryple Select, second passage cells are counted and resuspended in vials with culture medium. Cells are seeded in 40 µl doses on presterilized scaffolds in a dropwise fashion. Seeded constructs are placed for 2 h in the CO₂ incubator. The seeding of the cells enhances the cartilage restorative properties of the constructs, by allowing the cellular adhesion and subsequent proliferation on the constructs when in use in therapy. The remaining medium is added to support the effective cell growth and nutrient intake and metabolic waste disposal.

Seeded constructs are cultured up to 21 days. Medium is changed every 3-4 days. Constructs with cells are harvested after predetermined endpoints for pH, biocompatibility, type II collagen and TNF-a gene expression studies.

At every defined time interval, pH of the media is assessed with a pH meter.

### Drug loading and release:

The scaffolds are incubated in 5 ml of HBSS (ph = 7.2 - 7.3) at 37 °C in orbital shaker at 80 rpm. and quantified using a spectrophotometer at different time inter intervals up to 28 days. At different time points (2h, 4h, 8h, 24h, 2-day, 4-day, 8-day, 16-day, 21-day). 200 µl of the tube supernatant medium was withdrawn for spectrophotometric analysis and the medium replenished with 200 µl fresh HBSS. Samples were collected and read on a plate reader with the excitation wavelength set to 288 nm The absorbance of the total nicotinamide amount per respective scaffold was evaluated in the scaffold-less nicotinamide in the medium study. The resultant scaffold nicotinamide release absorbance values were normalized to the scaffold-less nicotinamide absorbance value in the medium and the cumulative amount of the drug released over time was calculated.

The nicotinamide release was evident throughout the 21-day observation period. The sustained release of the acid was evident in all groups tested. An increased release at 8 hour was evident in P/P/3 and P/P/6 groups, compared to P/P/1 (0.0194 and 0.0004, respectively). After 24 hours more nicotinamide was released in P/P/6 compared to P/P/1 (p<0.0001). The amount released to the medium positively correlated to the initial nicotinamide concentration per scaffold. Figure 1 shown the nicotinamide release kinetics. Statistical significances amongst the groups are shown in Table 1.

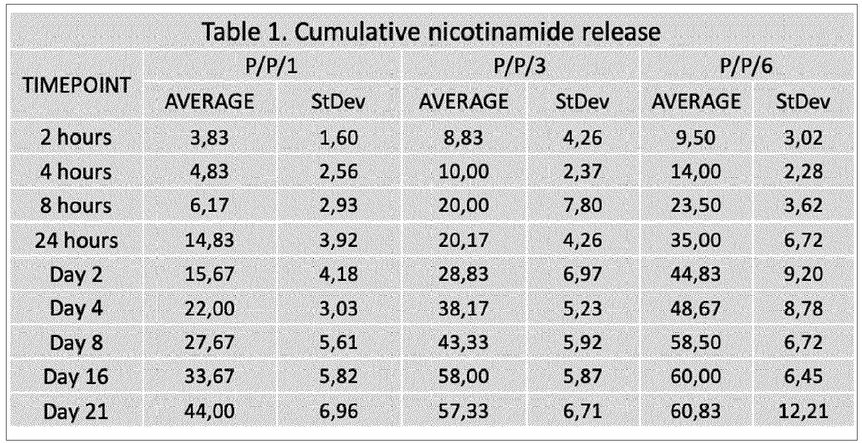

### Metabolic activity and cell count on PCL/PGLA scaffolds:

To evaluate *in vitro* metabolic activity on the PCL/PLGA nicotinamide loaded constructs, the cell viability of cultured perinatal stem cells on the constructs was observed by Presto Blue assay.

Briefly, scaffolds were placed in 24 well plates. A total of 1.5 × 10⁵ perinatal stem cells suspended in 20 µl of regular culture medium was added onto each scaffold in a drop-wise fashion and incubated for 2 h to ensure sufficient attachment of cells on the surface. After 2 h, 500 µl growth medium was added into each well of culture plate. Culture media were replaced with fresh growth media every fourth day. During the culture period, PSC proliferation was assessed by Presto Blue assay (1, 4 and 7 days). For the assay each construct was submerged in 10% Presto Blue solution and incubated for 30 min at 37 °C. Samples were collected and read on a plate reader with the excitation wavelength set to 570 nm. A standard curve of absorbance vs. metabolic activity ratio of samples and monolayer cells was plotted.

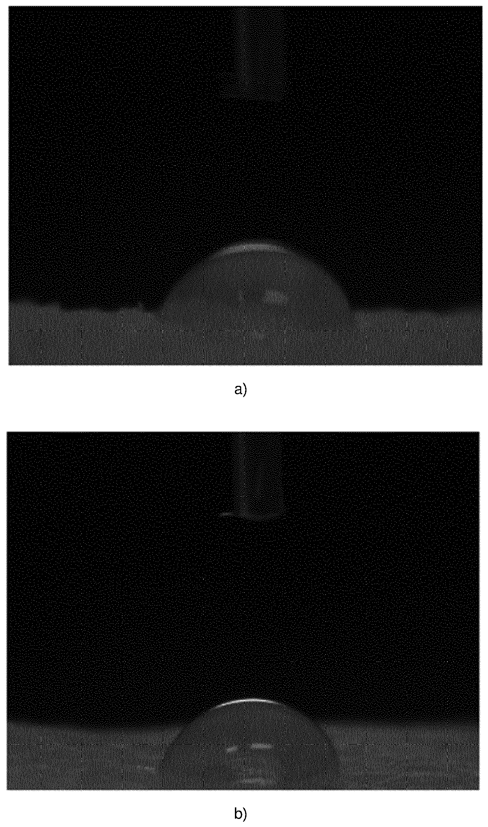

Cells seeded on all constructs improved their metabolic activity compared to cells used for seeding (monolayer cells) (Fig. 2). The metabolic activity improved in the all nicotinamide loaded groups. A comparable metabolic activity of the seeded 1% nicotinamide construct group was comparable to 3 % nicotinamide group up till day 7. However, the activity slightly dropped on day 7 in the 3 % group. 6% nicotinamide revealed a steady decline of metabolic activity of cells, suggesting toxic nicotinamide concentrations in this construct group. Statistical significances amongst the groups are shown in Table 2.

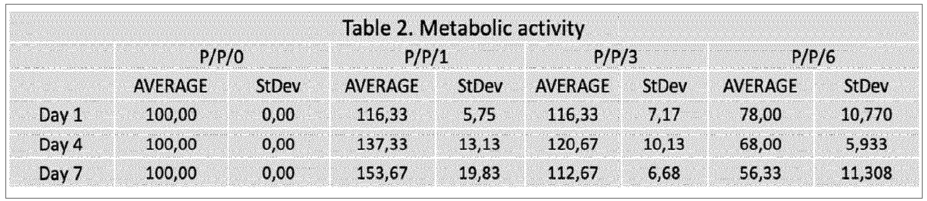

### Gene expression and protein secretion:

PBMCs method: to assess gene expression and protein secretion, peripheral blood mononuclear cells (PBMCs) were isolated by collecting 30 ml of venous blood. The collected blood was diluted with the same volume of PBS solution. Diluted blood was layered on top of Histopaque-1077 solution. After centrifugation, the PBMCs fraction was collected, washed, and centrifuged again. PBMCs were stained with CFSE and the basal fluorescence was measured. Constructs were seeded with 1.5 × 10⁵ perinatal mesenchymal stem cells in 20 µl of culture medium. Media was added after 2 hours, to enable the cell attachment to the constructs. After additional 2 hours, PBMCs are activated with PMA-Ionomycin solution (0.25 µg/ml - 0.5 µM) and added to the culture. After 24 hours of incubation supernatant was collected and aliquoted for subsequent analysis of TNF-alpha and IL-6 protein and gene expression (PCR study) and protein secretion (ELISA study). Activated, inactivated PBMCs and constructs without cells served as control groups.

PCR method: the total RNA of supernatants with PBMCs was extracted from the samples using an ISOLATE II RNA Micro Kit (Bioline, London, UK) according to the manufacturer's instructions. Designed primer pairs, probes, and the condition of amplification are represented in the supplementary data To analyze the data
obtained, the 2^{-ΔΔCT} method was applied for the relative gene expression data evaluation. GAPDH gene expression was used for data normalization.

ELISA method: Enzyme-Linked ImmunoSorbent Assay analyses TNF-alpha and IL-6 were performed following the manufacturer's (Biotechne, USA) instructions. The test was performed on 4 samples per condition, according to manufacturer's protocol. The optical density absorbance was read at 450 nm in a microplate reader (Multiskan GO, Thermo Scientific). A standard curve was plotted as the relative optical density of each standard solution versus the respective concentration of the standard solution. Respective genes concentration of the samples was interpolated from the standard curve.

To investigate the immunomodulatory effects of the PCL/PLGA loaded with nicotinamide constructs, expression and secretion of inflammatory markers were measured by RT-PCR and ELISA.

### PCR:

Expression of proinflammatory markers TNF-α and IL-6 beta was assessed after cultivating cell seeded constructs with peripheral blood mononuclear for 24 hours.

TNF-alpha gene results. PBMCs activation resulted in increased TNF-alpha expression (Fig. 3a). P/P/3 and P/P/6 had a superior diminishing TNF-alpha expression profile. The cell-untreated constructs witnessed diminished TNF gene expression profiles, whilst P/P/6 group had slightly higher values compared to other untreated constructs groups, albeit insignificantly. This suggests a diminished effect on TNF expression in P/P/6 constructs group.

Addition of cells significantly improved TNF expression suppressive capabilities in all groups tested. The cell treated groups were significantly superior compared to unloaded P/P/0 constructs (p<0.05 in all groups).

A clear tendency for the superior 3% loaded constructs amongst the groups was evident, as revealed by the gene expression and protein values. Statistical significances amongst the groups are shown in Table 3.

II-6 gene results. Similarly, PBMCs activation resulted in increased II-6 expression (Fig. 3b). All nicotinamide treated constructs groups diminished II-6 expression, and addition of cells only improved the untreated P/P/0 group, suggesting a combinatory positive effect of nicotinamide in other groups. Statistical significances amongst the groups are shown in Table 3.

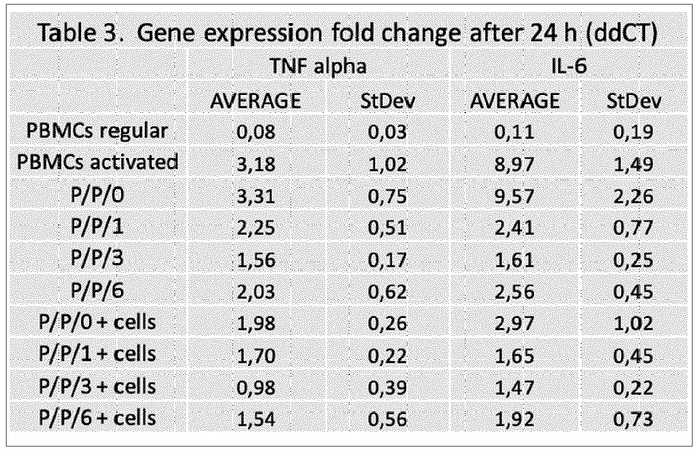

### ELISA:

Secretion of proinflammatory markers TNF-α and IL-6 beta was assessed after cultivating cell seeded constructs with peripheral blood mononuclear for 24 hours. Results correlated with the gene expression assays.

TNF-alpha protein results. Nicotinamide treated constructs had superior TNF-alpha protein secretion suppressing capabilities (p<0.0001), albeit untreated P/P/0 constructs did not have the same positive properties (Fig. 4a). However, addition of cells highly improved potency to diminish inflammation in all groups tested. Statistical significances amongst the groups are shown in Table 4.

II-6 protein results. Similarly, nicotinamide treated constructs had superior TNF-alpha protein secretion suppressing capabilities (p<0.0001), albeit, similarly to TNF-alpha protein analysis, untreated P/P/0 constructs did not have the same positive properties (Fig. 4b). Cells addition to the constructs significantly improved anti-inflammatory properties in P/P/0 and P/P/3 groups. P/P/6 had an inferior anti-inflammatory property and was not able to reduce protein secretion, compared to P/P/3 group (p=0.0115). Statistical significances amongst the groups are shown in Table 4.

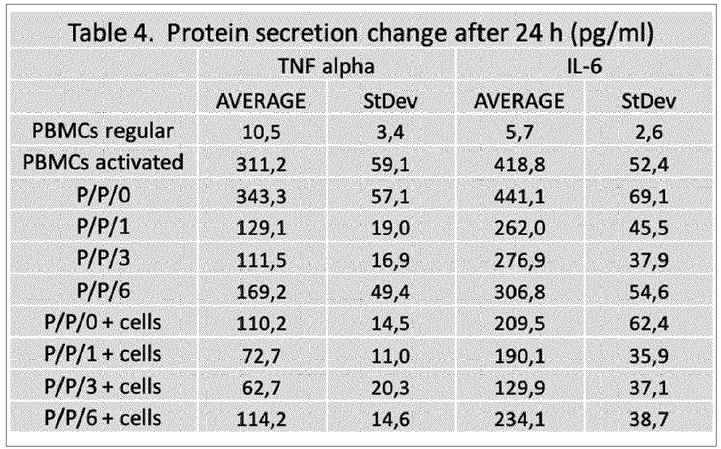

### References

1. Zhang L, Fu T, Zhang Q, Yin R, Zhu L, He Y, Fu W, Shen B. Effects of psychological interventions for patients with osteoarthritis: a systematic review and meta-analysis. Psychol Health Med. 2018 Jan;23(1):1-17. doi: 10.1080/13548506.2017.1282160. Epub 2017 Jan 31. PMID: 28140653.
2. Prakash Kumar, Bibaswan Dey, G. P. Raja Sekhar, Nutrient transport through deformable cylindrical scaffold inside a bioreactor: An application to tissue engineering, International Journal of Engineering Science, Volume 127, 2018, Pages 201-216, ISSN 0020-7225,
3. Elaheh Alizadeh, Jordan Castle, Analia Quirk, Cameron D.L. Taylor, Wenlong Xu, Ashok Prasad, Cellular morphological features are predictive markers of cancer cell state, Computers in Biology and Medicine, Volume 126, 2020, 104044,
4. Gao F, Chiu SM, Motan DA, Zhang Z, Chen L, Ji HL, Tse HF, Fu QL, Lian Q. Mesenchymal stem cells and immunomodulation: current status and future prospects. Cell Death Dis. 2016 Jan 21;7(1):e2062. doi: 10.1038/cddis.2015.327. PMID: 26794657; PMCID: PMC4816164.
5. Espinoza, S. M., Patil, H. I., San Martin Martinez, E., Casañas Pimentel, R., & Ige, P. P. (2020). Poly-ε-caprolactone (PCL), a promising polymer for pharmaceutical and biomedical applications: Focus on nanomedicine in cancer. International Journal of Polymeric Materials and Polymeric Biomaterials, 85-126.
6. Song R, Murphy M, Li C, Ting K, Soo C, Zheng Z. Current development of biodegradable polymeric materials for biomedical applications. Drug Des Devel Ther. 2018;12:3117-3145. Published 2018 Sep 24. doi:10.2147/DDDT.S165440
7. Ali Mobasheri, Gauthaman Kalamegam, Giuseppe Musumeci, Mark E. Batt, Chondrocyte and mesenchymal stem cell-based therapies for cartilage repair in osteoarthritis and related orthopaedic conditions, Maturitas, Volume 78, Issue 3, 2014, Pages 188-198,
8. Gnecchi M, Danieli P, Malpasso G, Ciuffreda MC. Paracrine Mechanisms of Mesenchymal Stem Cells in Tissue Repair. Methods Mol Biol. 2016;1416:123-46. doi: 10.1007/978-1-4939-3584-0_7. PMID: 27236669.
9. Soheila Zamanlui, Matin Mahmoudifard, Masoud Soleimani, Behnaz Bakhshandeh, Mohammad Vasei & Shahab Faghihi (2018) Enhanced chondrogenic differentiation of human bone marrow mesenchymal stem cells on PCL/PLGA electrospun with different alignments and compositions, International Journal of Polymeric Materials and Polymeric Biomaterials, 67:1, 50-60
10. Houchin M.; Topp E. Chemical degradation of peptides and proteins in PLGA: a review of reactions and mechanisms. J. Pharm. Sci. 2008, 97 (7), 2395.
11. S. Jin, X. Xia, J. Huang, C. Yuan, Y. Zuo, Y. Li, J. Li, Recent advances in PLGA-based biomaterials for bone tissue regeneration, Acta Biomater. 127 (2021) 56-79.

## Claims

**1.** Method for producing an anti-inflammatory three-dimensional cellular construct for cartilage regeneration, comprising electrospinning a scaffold **characterized by**
- use of polycaprolactone (PCL) material for making scaffold by electrospinning;
- selecting and using poly-D-L-lactide-co-glycolide (PLGA) with a lactic acid monomer content;
- addition of Nicotinamide particles to the scaffold;
wherein acetone and dimetilformamide solvents are used in preparation of polymer solution;
- lyophilizing by freezing the electrospun scaffold in a freezer preferably at -20°C for 24 h;
- lyophilizing frozen samples under a vacuum of 2 × 10⁻⁶ mPa at -40°C for 72 h;
- storing scaffold after lyophilization in glass airtight vials in the dark at 20-25°C and 40-60% relative humidity,
- adding mesenchymal stem cells.

**2.** Method according to claim 1, where poly-D-L-lactide-co-glycolide (PLGA) with a lactic acid monomer content is of 75% in the polymer and a glycolic acid monomer content of 25%, maintaining a monomer ratio of 75:25.

**3.** Method according to claim 1 or 2 wherein Nicotinamide concentration is 0-6%, preferably - 1%.

**4.** Method according to any one claims of 1 - 3, wherein polycaprolactone (PCL) of mol wt 80,000 and PLGA- Poly(D,L-lactide-co-glycolide) lactide:glycolide (ratio 75:25), mol wt 66,000-107,000 are used.

**5.** Method according to any one claims of 1 - 4, wherein acetone and dimetilformamide solvents are used in preparation of polymer solution having concentration at 20% wt/v.

**6.** Method according to any one claims of 1 - 6, wherein solvent ratio of acetone:dimetilformamide is 2:3 v/v.

**7.** Method according to any one claims of 1 - 6, wherein electrospinning parameters for electrospinning a scaffold according to the invention are as follows:
- polymer solution supply flow 2.3 ml/h;
- needle no. 21;
- distance between needle and collector 15 cm;
- voltage between needle and collector 22 kV;
- ambient temperature 30°C;
- relative humidity 40%;
- using Aluminum foil substrate;
- the needle moves in the x-axis at 5 cm intervals;
- the polymer solution is supplied with a Teflon hose;
- adding drying ice to the collector every 25 minutes.

**8.** A bioactive anti-inflammatory three-dimensional cellular construct for cartilage regeneration, produced by method according to any one of claims 1-7, comprising electrospun filaments, **characterized in that** it comprises polycaprolactone, poly-D-L-lactide-co-glycolide Nicotinamide acid, and mesenchymal stem cells seeded onto the scaffold.

**9.** Construct according to claim 8, where the construct comprises PCL / PLGA 3/1 (wt / wt) + Nicotinic acid 1% (By weight of PLGA).

**11.** Construct according to claim 8, where the construct comprises PCL / PLGA 3/1 (wt / wt) + Nicotinic acid 3% (By weight of PLGA).

**12.** Construct according to claim 8, where the construct comprises PCL / PLGA 3/1 (wt / wt) + Nicotinic acid 1% (By weight of PLGA) 6%
